# EUROPEAN PATENT APPLICATION

(11) **EP 4 163 385 A1**
(43) Date of publication of application: **12.04.2023**
(21) Application number: 20938632.5
(22) Date of filing: 03.06.2020
(51) Int. Cl.: C12Q 1/68, G16H 50/00, G16B 40/00

(54) **DIAGNOSIS SUPPORT PROGRAM, DEVICE, AND METHOD**

(71) Applicant: FUJITSU LIMITED, Kawasaki-shi, Kanagawa 211-8588 (JP)
(72) Inventor: YANASE, Takashi, Kawasaki-shi, Kanagawa 211-8588 (JP)
(74) Representative: Hoffmann Eitle
(86) International application number: PCT/JP2020/021994
(87) International publication number: WO 2021/245850

(57) **Abstract**

A diagnosis support device supports determination of a diagnostic criterion effective for diagnosis, in a case of using machine learning for the determination of the diagnostic criterion, by generating a training data set obtained by binarizing gene expression levels of sample data, exhaustively enumerating hypotheses configured by combinations of all features, performing machine learning for training data using an AI capable of assigning the degree of importance to the hypotheses, acquiring a rule set in which each rule is represented by each of the exhaustive combinations of a plurality of genes, and is associated with a rule weight leading to a hypothesis of the presence of a disease, determining a pattern weight based on the rule weight for each pattern including types of a predetermined number of genes, and outputting the pattern with the pattern weight that is equal to or greater than a predetermined value as a diagnostic criterion candidate.

## Description

### FIELD

The technology disclosed herein relates to a diagnosis support program, a diagnosis support device, and a diagnosis support method.

### BACKGROUND

In a medical setting or the like, the presence or absence of a disease is diagnosed based on a feature indicated by a sample collected from a patient and a predetermined diagnostic criterion. As a method of determining the diagnostic criterion, for example, there is a determination method by creating a model for predicting the presence or absence of a disease to be diagnosed. The model is created by machine learning such as a support vector machine (SVM) or a decision tree, using training data that associates the feature indicated by the sample collected from each of patients with and without the disease to be diagnosed with a classification label that indicates the presence or absence of the disease.

For example, a method of stratifying a subject according to an event occurring in the subject's body has been proposed. In this method, for a population of biomarkers derived from a subject, whether each biomarker varies in relation to an event occurring in the body of the subject based on a measured value of each biomarker or not is determined by a statistical method. Then, a biomarker group determined to vary is extracted as a first subpopulation. Furthermore, each biomarker belonging to the first subpopulation is verified, and a biomarker group statistically predicted to have a stronger relationship with an event occurring in the body is extracted as a second subpopulation. Then, a weight of each biomarker belonging to the second subpopulation is calculated by a deep learning method and a discriminator is generated. The discriminator calculates a weighted sum of scores of the biomarkers belonging to the second subpopulation, using a score obtained from the measured value of each biomarker belonging to the second subpopulation and the calculated weight of each biomarker.

### CITATION LIST

### PATENT DOCUMENT

Patent Document 1: Japanese Laid-open Patent Publication No. 2020-28278

### SUMMARY

### [TECHNICAL PROBLEM]

In a case where the number of types of features used as explanatory variables for machine learning is enormous, it is difficult to create a model using conventional machine learning. For example, in a case where the feature is an expression level of a gene, the number of gene types may be 10,000 or more. It is conceivable to perform machine learning after selecting features to be used as explanatory variables, such as narrowing down genes to only genes that are likely to be effective in disease prediction, as in the existing technology. However, in this case, many types of features are excluded from the explanatory variables, and the features that are excluded may include features intrinsically effective for diagnosis. For example, even in a case where a feature alone is less effective in predicting disease, it may be more effective in combination with another feature, but such a feature may be excluded from the explanatory variables. As a result, the diagnostic criterion for effectively performing diagnosis may not be able to be determined.

As one aspect, the technology disclosed herein aims to support determination of a diagnostic criterion effective for diagnosis in a case of using machine learning for determining the diagnostic criterion.

### [SOLUTION TO PROBLEM]

As one aspect, the disclosed technology acquires a set of rules represented by a combination of one or more features, and generated by machine learning using a training data set including a feature indicated by a sample as a diagnosis target and a feature indicated by a sample other than the diagnosis target. Each of the rules is associated with a first weight for the diagnosis target. Furthermore, the disclosed technology determines, for each pattern including a predetermined number of features, a second weight based on the first weight associated with the rule including the feature included in the pattern, and outputs the pattern with the determined second weight that is equal to or greater than a predetermined value.

According to an aspect of the embodiments, a diagnosis support program that causes a computer to execute a process, the process includes acquiring a set of rules, each rule represented by a combination of one or more features and generated by machine learning by using a training data set that includes a feature indicated by a sample as a diagnosis target and a feature indicated by a sample as a non-diagnosis target, and the each rule being associated with a first weight for the diagnosis target; determining, for each pattern that includes a predetermined number of features, a second weight based on the first weight associated with the rule that includes the feature included in the pattern; and outputting the pattern with the determined second weight that is equal to or greater than a predetermined value.

### [ADVANTAGEOUS EFFECTS OF INVENTION]

As one aspect, in a case of using machine learning in determining a diagnostic criterion, an effect of supporting effective determination of the diagnostic criterion is exhibited.

### BRIEF DESCRIPTION OF DRAWINGS

FIG. 1 is a functional block diagram of a diagnosis support device;
FIG. 2 is a table illustrating an example of a sample data set;
FIG. 3 is tables for describing a case of performing machine learning by narrowing down features to be used as explanatory variables for machine learning;
FIG. 4 is tables for describing generation of a training data set;
FIG. 5 is a table illustrating an example of a rule set;
FIG. 6 is tables for describing generation of patterns;
FIG. 7 is a table for describing an example of pattern weight correction;
FIG. 8 is a diagram illustrating an example of an output screen of diagnostic criterion candidates;
FIG. 9 is a block diagram illustrating a schematic configuration of a computer that functions as the diagnosis support device;
FIG. 10 is a flowchart illustrating an example of diagnosis support processing;
FIG. 11 is a flowchart illustrating an example of training data generation processing;
FIG. 12 is a flowchart illustrating an example of rule acquisition processing;
FIG. 13 is a flowchart illustrating an example of pattern generation processing;
FIG. 14 is a flowchart illustrating an example of weight correction processing; and
FIG. 15 is a schematic diagram for describing diagnosis support processing.

### DESCRIPTION OF EMBODIMENTS

Hereinafter, an example of embodiments according to the disclosed technology will be described with reference to the drawings. In the following embodiments, a case of supporting determination of a diagnostic criterion used for genetic diagnosis will be described. The genetic diagnosis is a method of diagnosing the presence or absence of a disease by examining whether a specific gene is expressed in a tissue sample collected from a patient. Therefore, as the diagnostic criterion, a type of a gene that is highly expressed in the presence of a disease is determined.

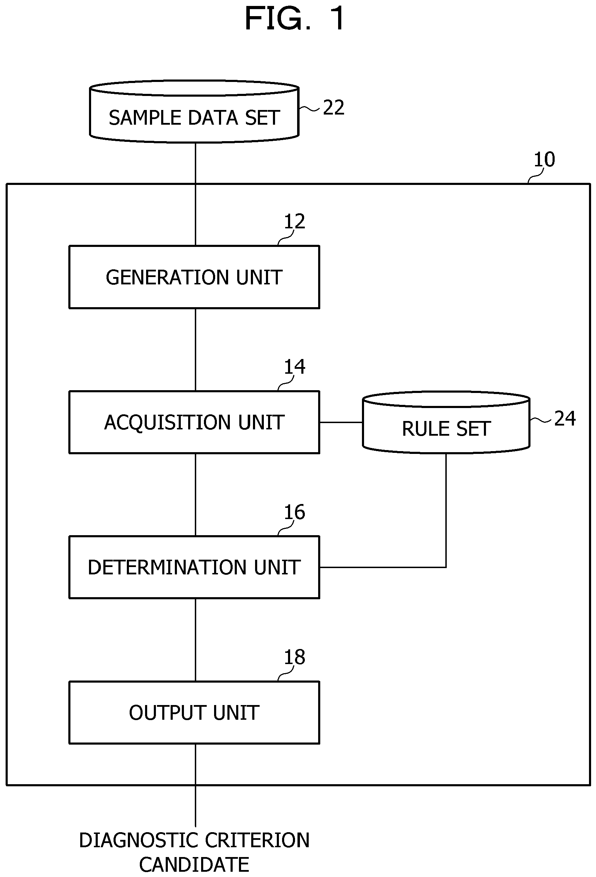

As illustrated in FIG. 1, a sample data set 22 is input to a diagnosis support device 10. The diagnosis support device 10 performs machine learning for a training data set generated from the sample data set 22 to extract and output diagnostic criterion candidates as described above.

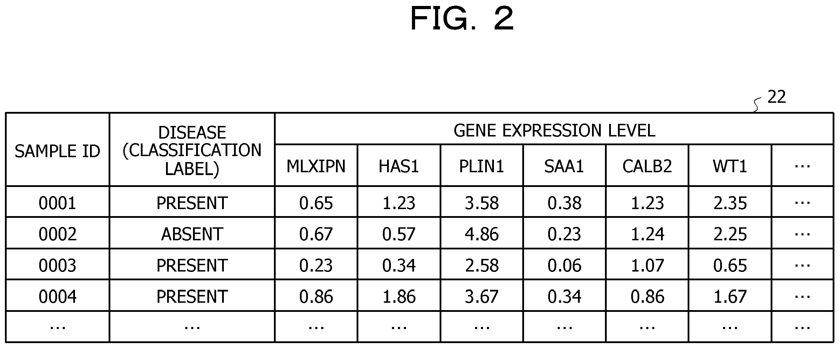

The sample data set 22 is a set of sample data that is data of an expression level for each of a plurality of types of genes extracted from tissue samples collected from patients with and without a disease to be diagnosed. FIG. 2 illustrates an example of the sample data set 22. In the example of FIG. 2, each row (each record) corresponds to one sample data. In the example of FIG. 2, each sample data is given a "sample ID" that is identification information of the sample data. Furthermore, each sample data is associated with a "disease (classification label)" indicating whether the patient corresponding to the sample data has a disease to be diagnosed or does not have the disease. Furthermore, each sample data includes, for each type of gene, information of the expression level of the gene ("gene expression level" in FIG. 2) extracted from the sample data.

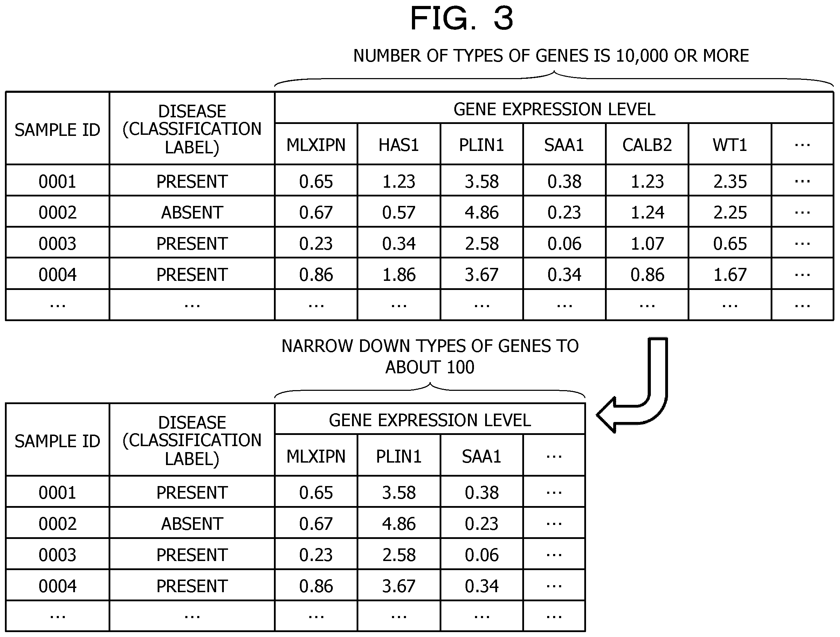

Here, as illustrated in FIG. 3, consider a case of narrowing down features to be used as explanatory variables for machine learning and performing the machine learning. The example in FIG. 3 illustrates a case where more than 10,000 types of genes included in sample data are narrowed down to about 100 and used as training data. The narrowing down of the types of genes is determined based on, for example, a correlation of expression levels between genes or the like. In the example of FIG. 3, the diagnostic criterion is determined by a model created by performing machine learning for a training data set in which genes such as HAS1, CALB2, and WT1 are excluded from the sample data. In this case, even if the excluded genes HAS1, CALB2, WT1, and the like are diagnostically effective, these excluded genes are not included in the diagnostic criterion.

Therefore, according to the present embodiment, in the machine learning, artificial intelligence (AI) having characteristics such as "capable of explaining reasons for evaluation", "exhaustively enumerating hypotheses configured by combinations of all variables (features)", and "capable of assigning degrees of importance to these hypotheses" is applied.

The diagnosis support device 10 functionally includes a generation unit 12, an acquisition unit 14, a determination unit 16, and an output unit 18, as illustrated in FIG. 1.

The generation unit 12 generates the training data set to be used for the machine learning for extracting the diagnostic criterion candidates from the sample data set 22 input to the diagnosis support device 10. Specifically, the generation unit 12 converts the gene expression level of the sample data included in the sample data set 22 into a binary value indicating high expression or low expression.

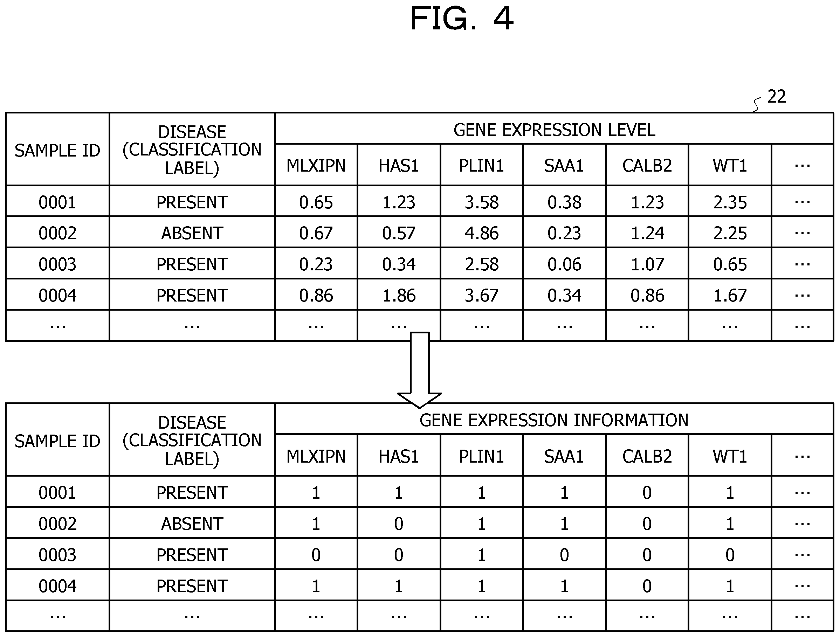

For example, the generation unit 12 determines a threshold for each type of gene using an existing binarization method. Examples of the existing binarization methods include a dynamic threshold method used in image binarization and the like, and a step-minor method used in the field of genetics. Then, as illustrated in FIG. 4, the generation unit 12 converts the gene expression level into a value indicating high expression (for example, "1") in a case where the gene expression level is greater than the threshold. On the other hand, the generation unit 12 converts the gene expression level into a value indicating low expression (for example, "0") in a case where the gene expression level is equal to or less than the threshold.

The generation unit 12 generates training data by binarizing the gene expression level of the sample data as described above. In other words, the training data set is a set of training data in which a binarized value of each of the gene expression levels is associated with the classification label. Below, the binarized gene expression level is referred to as "gene expression information". The lower table in FIG. 4 represents the training data set, and each row (each record) corresponds to one piece of training data. The generation unit 12 passes the generated training data set to the acquisition unit 14.

The acquisition unit 14 acquires a set of rules, each rule being represented by a combination of one or more features and generated by machine learning using the training data set passed from the generation unit 12, and the each rule being associated with a weight for a diagnosis target.

Specifically, the acquisition unit 14 applies the AI having the above-described characteristics and performs the machine learning for the training data using the gene expression information as explanatory variables and the classification labels as objective variables. Therefore, the acquisition unit 14 acquires, as a rule, a hypothesis leading to a diagnosis of the presence of the disease to be diagnosed. More specifically, the AI applied in the present embodiment exhaustively enumerates combinations of a plurality of types of genes. Then, the AI calculates, for each combination, the degree of contribution (degree of importance) of the high expression of the genes included in the combination to the diagnosis result of the presence of the disease to be diagnosed from the association of the gene expression information of the training data with the classification label by the machine learning. In other words, the combination of highly expressed genes explains why the presence of the disease to be diagnosed is diagnosed. Furthermore, by using the training data obtained by binarizing the gene expression levels, efficient machine learning can be performed for each of the exhaustive combinations of types of genes.

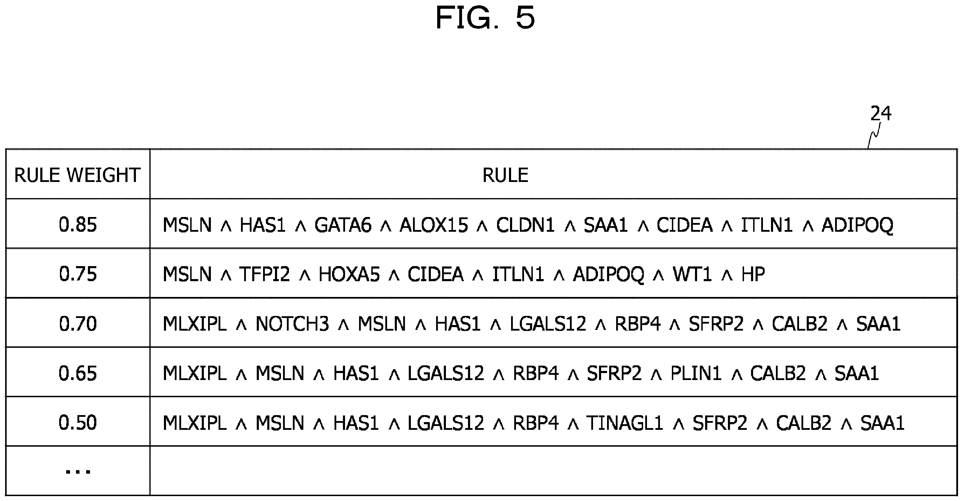

The acquisition unit 14 acquires the combination of highly expressed genes as a rule and the degree of importance assigned to the rule as a rule weight, and stores the rule and the rule weight in a predetermined storage area as a rule set 24 as illustrated in FIG. 5. The rule weight is an example of a "first weight" of the disclosed technology. Note that the acquisition unit 14 may include only rules with the rule weights that are equal to or greater than a predetermined value in the rule set 24.

The determination unit 16 determines, for each pattern including types of predetermined number of genes, a pattern weight based on the rule weight associated with the rule including the types of genes included in the pattern. In a case where the predetermined number is multiple, in other words, in a case where a plurality of types of genes is included in the pattern, a combination of genes that are related to the disease to be diagnosed in a case where a plurality of genes is co-expressed can be extracted as a diagnostic criterion candidate.

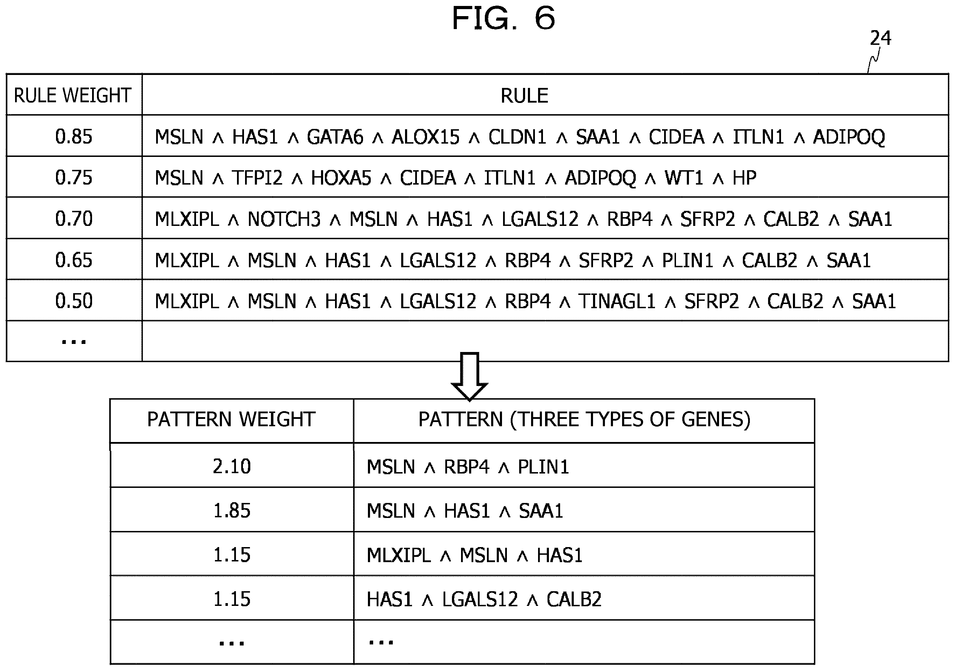

Specifically, the determination unit 16 receives, from a user, specification of the number of types of genes to be included in the pattern, and generates a combination of the genes of the specified number of types (three types in the example of FIG. 6) as a pattern, as illustrated in FIG. 6. The determination unit 16 searches the rule set 24 for a rule that includes all the types of genes included in the pattern, for each generated pattern. Then, the determination unit 16 calculates a total value of the rule weights associated with the searched rules as the pattern weight. Therefore, it is possible to calculate a larger rule weight as the degree of conformity to the hypothesis leading to the diagnosis of the presence of the disease to be diagnosed is higher. Note that the method of calculating the rule weight is not limited to the above example, and may be the product, weighted sum, average, or the like of the rule weights associated with the searched rule.

Furthermore, in a case where the pattern includes a gene with an unknown function and a gene with a known function and related to the disease to be diagnosed, the determination unit 16 corrects the calculated pattern weight. Specifically, the determination unit 16 corrects the pattern weight to become larger as the number or ratio of genes with unknown function included in the pattern is larger. This is intended to support discovery of a new diagnostic criterion involving genes with unknown functions. Furthermore, the reason for correcting the pattern weight to be large in the case where a gene with an unknown function is included in the pattern together with a gene with a known function and related to the disease to be diagnosed is that there is no basis for the gene with an unknown function alone to be associated with the disease.

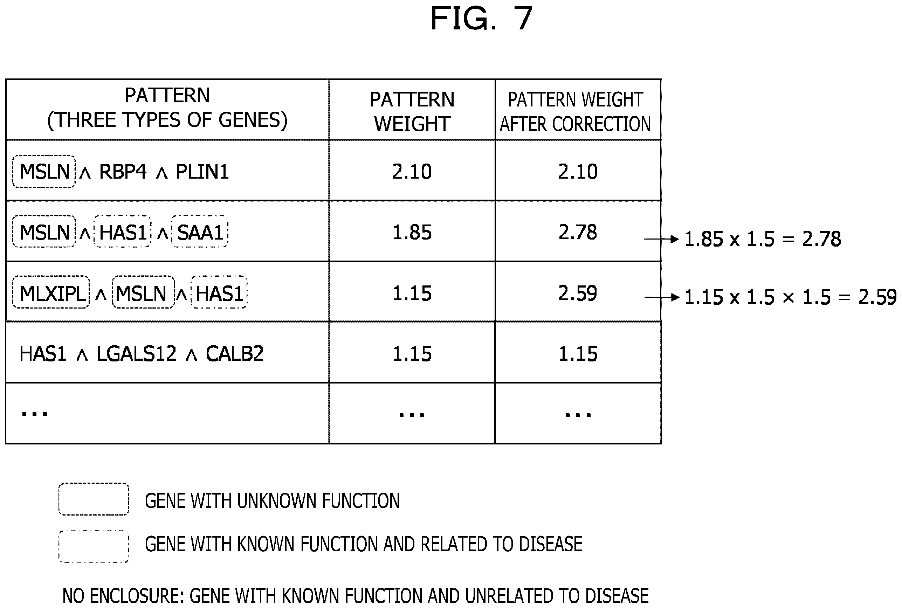

FIG. 7 illustrates an example of pattern weight correction. For example, for the pattern including a gene with an unknown function and a gene with a known function and related to the disease to be diagnosed, the determination unit 16 multiplies the calculated pattern weight by 1.5 once for one gene with an unknown function included in the pattern. Note that the pattern weight correction method is not limited to this, and may be corrected by other methods such as adding a value corresponding to the number or ratio of genes with unknown functions.

The determination unit 16 determines the corrected pattern weight as the final pattern weight, and passes the pattern and the pattern weight to the output unit 18. Note that the pattern weight is an example of a "second weight" of the disclosed technology.

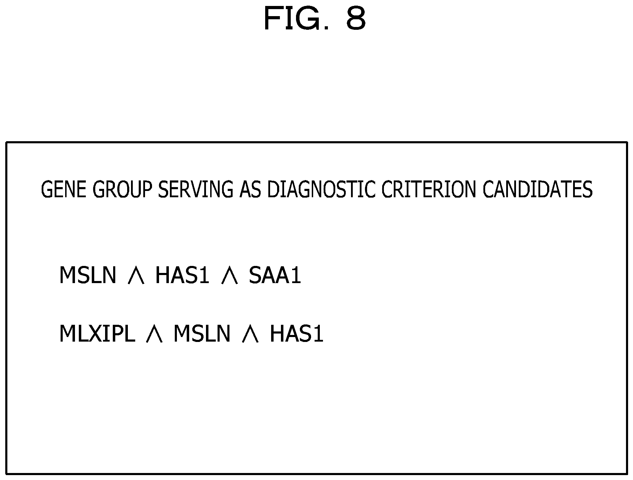

The output unit 18 outputs the genes included in the pattern in which the pattern weight determined by the determination unit 16 is equal to or greater than a predetermined value, as a gene group that serves as a diagnostic criterion candidate. The output information is displayed on an output screen on, for example, a display of an information processing terminal used by a doctor or the like, as illustrated in FIG. 8. The example of FIGs. 7 and 8 illustrates an example in which the patterns with the pattern weight of 2.5 or higher are output as the gene groups that serve as the diagnostic criterion candidates. Note that the information of the gene groups that serve as the diagnostic criterion candidates is not limited to being displayed on a display, and may be output by other methods such as being printed out on paper.

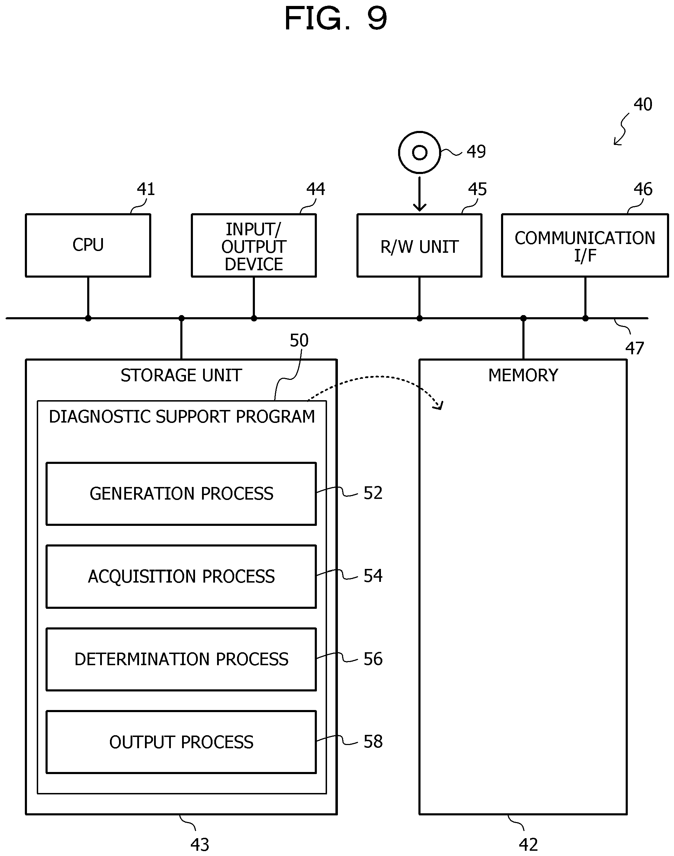

The diagnosis support device 10 can be implemented by a computer 40 illustrated in FIG. 9, for example. The computer 40 includes a central processing unit (CPU) 41, a memory 42 as a temporary storage area, and a nonvolatile storage unit 43. Furthermore, the computer 40 includes an input/output device 44 such as an input unit and a display unit, and a read/write (R/W) unit 45 that controls reading and writing of data from/to a storage medium 49. Furthermore, the computer 40 includes a communication interface (I/F) 46 to be connected to a network such as the Internet. The CPU 41, the memory 42, the storage unit 43, the input/output device 44, the R/W unit 45, and the communication I/F 46 are connected to each other via a bus 47.

The storage unit 43 may be implemented by a hard disk drive (HDD), a solid state drive (SSD), a flash memory, or the like. A diagnosis support program 50 for causing the computer 40 to function as the diagnosis support device 10 is stored in the storage unit 43 as a storage medium. The diagnosis support program 50 has a generation process 52, an acquisition process 54, a determination process 56 and an output process 58.

The CPU 41 reads out the diagnosis support program 50 from the storage unit 43, expands the diagnosis support program 50 in the memory 42, and sequentially executes processes included in the diagnosis support program 50. The CPU 41 executes the generation process 52 to operate as the generation unit 12 illustrated in FIG. 1. Furthermore, the CPU 41 executes the acquisition process 54 to operate as the acquisition unit 14 illustrated in FIG. 1. Furthermore, the CPU 41 executes the determination process 56 to operate as the determination unit 16 illustrated in FIG. 1. Furthermore, the CPU 41 operates as the output unit 18 illustrated in FIG. 1 by executing the output process 58. Furthermore, the CPU 41 expands the rule set 24 in the memory 42 when executing the acquisition process 54. Therefore, the computer 40 that has executed the diagnosis support program 50 functions as the diagnosis support device 10. Note that the CPU 41 that executes programs is hardware.

Note that the functions implemented by the diagnosis support program 50 can also be implemented by, for example, a semiconductor integrated circuit, in more detail, an application specific integrated circuit (ASIC) or the like.

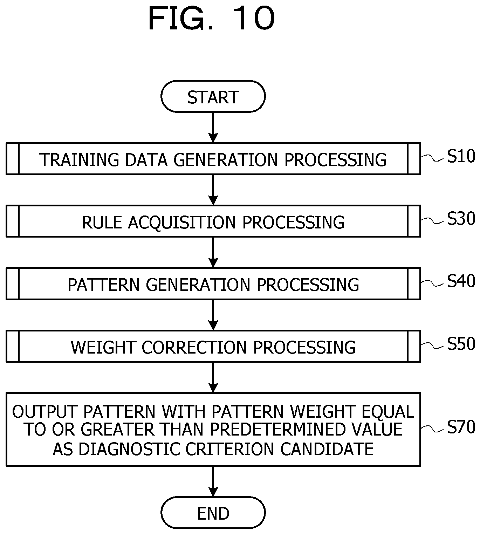

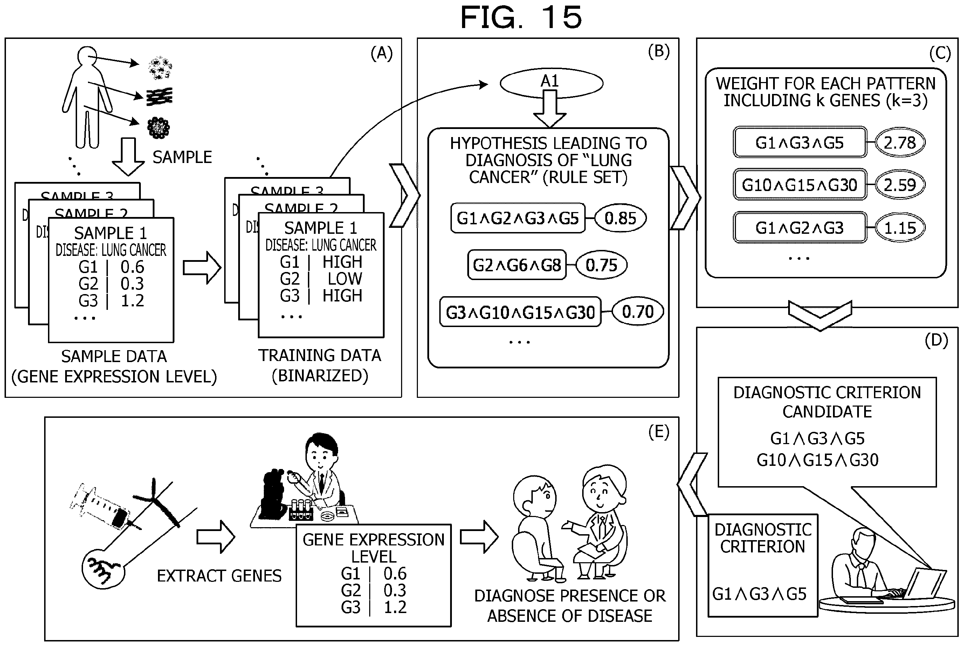

Next, operation of the diagnosis support device 10 according to the present embodiment will be described. When the sample data set 22 is input to the diagnosis support device 10, diagnosis support processing illustrated in FIG. 10 is executed in the diagnosis support device 10. Note that the diagnosis support processing is an example of a diagnosis support method of the disclosed technology. Hereinafter, a flowchart illustrating an example of the diagnosis support processing in FIG. 10 will be described with reference to the schematic diagram of the diagnosis support processing illustrated in FIG. 15 as well.

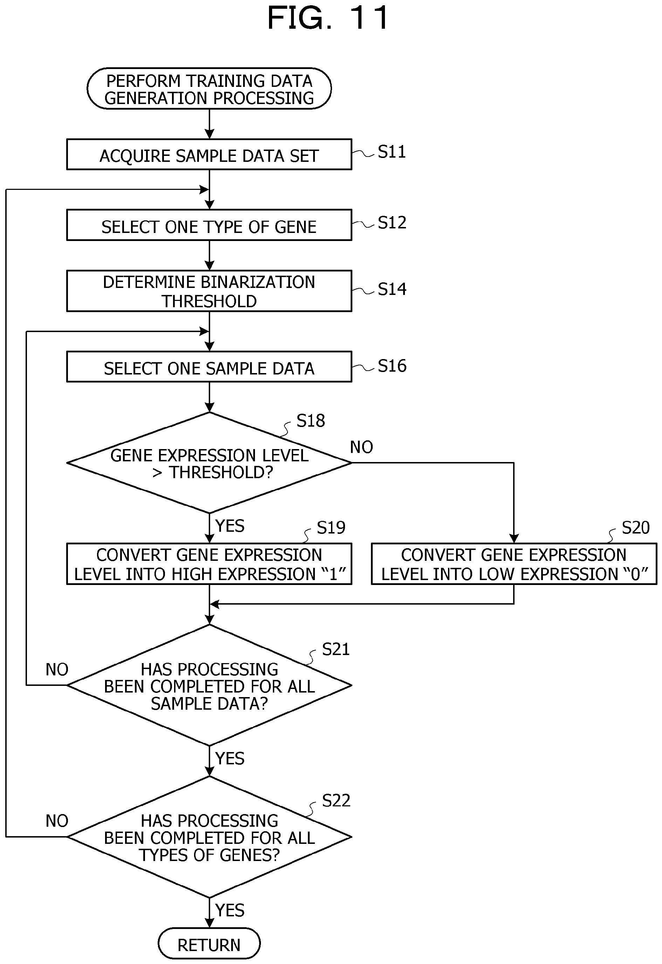

In step S10, the generation unit 12 executes training data generation processing. Here, the training data generation processing will be described with reference to FIG. 11.

In step S11, the generation unit 12 acquires the sample data set 22 input to the diagnosis support device 10. Next, in step S12, the generation unit 12 selects one type of gene for which the following processing has not been done yet from among the types of genes included in the sample data set 22. Next, in step S14, the generation unit 12 determines a binarization threshold for the selected type of gene by an existing binarization method.

Next, in step S16, the generation unit 12 selects one sample data for which the following processing has not been done yet from the sample data set 22. Next, in step S18, the generation unit 12 determines whether the gene expression level of the selected type of gene in the selected sample data is larger than the determined threshold or not. In a case of the gene expression level > the threshold, the processing proceeds to step S19, and in a case of the gene expression level ≤ the threshold, the processing proceeds to step S20.

In step S19, the generation unit 12 converts the gene expression level into a value (for example, "1") indicating high expression. Meanwhile, in step S20, the generation unit 12 converts the gene expression level into a value indicating low expression (for example, "0").

Next, in step S21, the generation unit 12 determines whether the processing of the above steps S18 to S20 has been completed or not for all the sample data included in the sample data set 22. In a case where unfinished sample data is present, the processing returns to step S16, or in a case where the processing has been completed for all the sample data, the processing proceeds to step S22.

In step S22, the generation unit 12 determines whether the processing of the above steps S14 to S21 has been completed or not for all the types of genes. In a case where an unfinished type of gene is present, the processing returns to step S12, or in a case where the processing has been completed for all the types of genes, the training data generation processing ends and the processing returns to the diagnosis support processing (FIG. 10). Therefore, the training data set in which the gene expression level of the sample data is binarized is generated, as illustrated in (A) of FIG. 15.

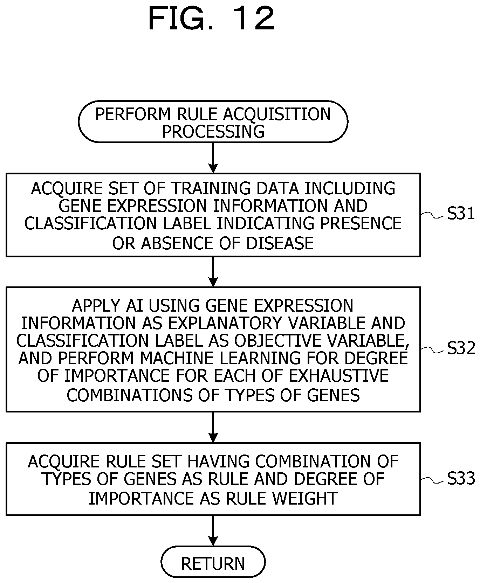

Next, in step S30, the acquisition unit 14 executes rule acquisition processing. Here, the rule acquisition processing will be described with reference to FIG. 12.

In step S31, the acquisition unit 14 acquires the training data set generated by the generation unit 12. Each training data included in the training data set includes the gene expression information and the classification label indicating the presence or absence of a disease.

Next, in step S32, the acquisition unit 14 performs machine learning for the training data, applying the AI having the above-described characteristics, and using the gene expression information as the explanatory variables and the classification labels as the objective variables. Specifically, the acquisition unit 14 causes the AI to exhaustively enumerate combinations of a plurality of types of genes. Then, the acquisition unit 14 causes the AI to calculate, for each combination, the degree of contribution (degree of importance) of the high expression of the genes included in the combination to the diagnosis result of the presence of the disease to be diagnosed from the association of the gene expression information of the training data with the classification label by the machine learning.

Next, in step S33, the acquisition unit 14 acquires the combination of highly expressed genes as a rule and the degree of importance assigned to the rule as a rule weight, and stores the rule and the rule weight in a predetermined storage area as a rule set 24. Then, the rule acquisition processing ends, and the processing returns to the diagnosis support processing (FIG. 10). Therefore, the acquisition unit 14 acquires the rule indicating the hypothesis leading to the diagnosis of the presence of the disease to be diagnosed ("lung cancer" in the example of FIG. 15) and the rule weight as the rule set, as illustrated in (B) of FIG. 15.

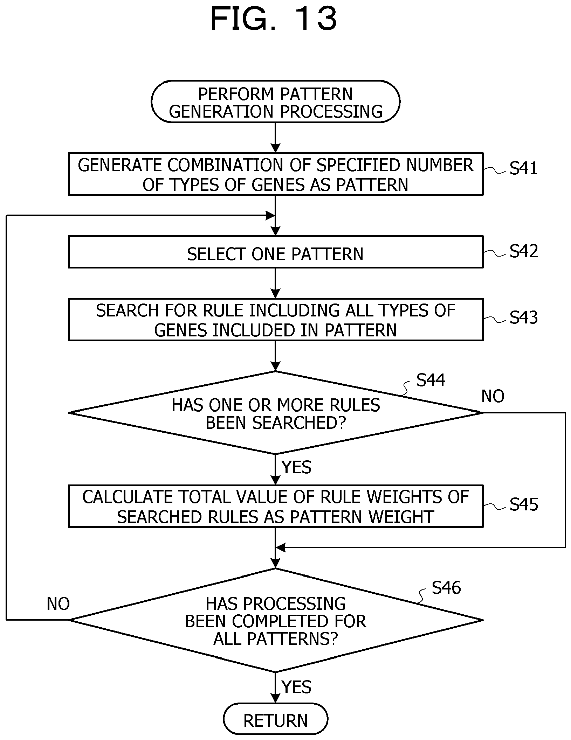

Next, in step S40, the determination unit 16 executes pattern generation processing. Here, the pattern generation processing will be described with reference to FIG. 13.

In step S41, the determination unit 16 receives specification of the number of types of genes to be included in the pattern from the user, and generates combinations of the genes of the specified number of types as patterns. Next, in step S42, the determination unit 16 selects one pattern for which the following processing has not been done yet from the generated patterns.

Next, in step S43, the determination unit 16 searches the rule set 24 for the rule that includes all the types of genes included in the selected pattern. Next, in step S44, the determination unit 16 determines whether one or more rules have been searched in the above step S43. In a case where one or more rules have been searched, the processing proceeds to step S45, and in a case where any rule has not been searched, the processing proceeds to step S46.

In step S45, the determination unit 16 calculates the total value of the rule weights associated with the searched rules as the pattern weight of the selected pattern. Next, in step S46, the determination unit 16 determines whether the processing of the above steps S43 to S45 has been completed for all the generated patterns or not. In a case where an unfinished pattern is present, the processing returns to step S42, or in a case where the processing has been completed for all the patterns, the pattern generation processing ends and the processing returns to the diagnosis support processing (FIG. 10).

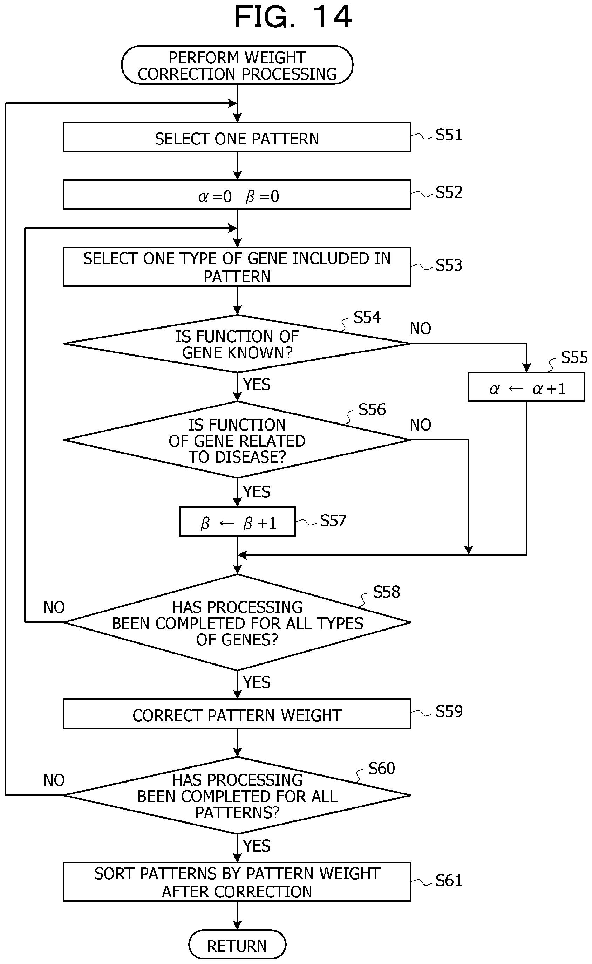

Next, in step S50, the determination unit 16 executes weight correction processing. Here, the weight correction processing will be described with reference to FIG. 14.

In step S51, the determination unit 16 selects one pattern generated by the pattern generation processing. Next, in step S52, the determination unit 16 sets 0 for a variable α for counting the number of types of genes with unknown functions, and sets 0 for a variable β for counting the number of types of genes with known functions and related to the disease to be diagnosed, included in the pattern.

Next, in step S53, the determination unit 16 selects one unprocessed type of gene from among the types of genes included in the selected pattern. Next, in step S54, the determination unit 16 determines whether the gene of selected type is a gene with a known function or not. In a case of a gene with a known function, the processing proceeds to step S56. On the other hand, in a case of a gene with an unknown function, the processing proceeds to step S55, the determination unit 16 increments α by 1, and the processing proceeds to step S58.

In step S56, the determination unit 16 determines whether the selected type of gene is related to the disease to be diagnosed or not. In a case of the gene related to the disease, the processing proceeds to step S57, and in a case of the gene unrelated to the disease, the processing proceeds to step S58. In step S57, the determination unit 16 increments β by 1, and the processing proceeds to step S58.

In step S58, the determination unit 16 determines whether the processing of the above steps S53 to S57 has been completed for all the types of genes included in the selected pattern or not. In a case where an unfinished type of gene is present, the processing returns to step S53, or in a case where the processing has been completed for all the types of genes, the processing proceeds to step S59.

In step S59, the determination unit 16 corrects the pattern weight of the selected pattern based on α and β. Specifically, in a case of α > 0 and β > 0, the determination unit 16 corrects the pattern weight to become larger as the number or ratio of α is larger. For example, the determination unit 16 corrects the pattern weight as follows: "the pattern weight before correction × γα (γ is a constant, for example, 1.5)".

Next, in step S60, whether the processing of the above steps S52 to S59 has been completed for all the patterns or not is determined. In a case where an unfinished pattern is present, the processing returns to step S51, or in a case where the processing has been completed for all the patterns, the processing proceeds to step S61. In step S61, the determination unit 16 determines the pattern weight after correction as the final pattern weight, and sorts the patterns in descending order of the pattern weight. Then, the weight correction processing ends, and the processing returns to the diagnosis support processing (FIG. 10).

By the pattern generation processing and the weight correction processing, the pattern weight is determined based on the rule weight for each pattern including types of predetermined number (k = 3 in the example of FIG. 15) of genes, as illustrated in (C) of FIG. 15.

Next, in step S70, the output unit 18 outputs the genes included in the pattern in which the pattern weight determined by the determination unit 16 is equal to or greater than a predetermined value, as the gene group that serves as the diagnostic criterion candidate. The predetermined value may be a value determined in advance, or may be the value of the top N-th pattern weight. In the latter case, the patterns with the top N pattern weights are output as the diagnostic criterion candidates.

Therefore, a doctor or the like refers to the output gene group that serves as a diagnostic criterion candidate and determines the diagnostic criterion as the gene to be tested based on medical knowledge, as illustrated in (D) of FIG. 15. Then, in the genetic diagnosis scene, as illustrated in (E) of FIG. 15, for example, blood is collected from the patient, the expression level of the gene to be tested indicated by the diagnostic criterion is measured, and the presence or absence of the disease is determined based on a measurement result.

As described above, the diagnosis support device according to the present embodiment acquires a set of rules, each rule being represented by a combination of one or more types of genes and generated by machine learning, and the each rule being associated with the rule weight for the disease to be diagnosed. The rule is created by performing machine learning for the gene expression information with the presence and absence of the disease, applying the AI that assigns the degree of importance according to the degree of contribution to the diagnosis result in the case where the genes included in the combination are highly expressed, for each exhaustive combination of genes. The diagnosis support device determines, for each pattern including types of predetermined number of genes, a pattern weight based on the rule weight associated with the rule including the types of genes included in the pattern, and outputs the pattern with the determined pattern weight that is equal to or greater than a predetermined value as the diagnostic criterion candidate. Therefore, it is possible to support determination of the diagnostic criterion effective for diagnosis in the case of using machine learning for determining the diagnostic criterion.

Furthermore, in the case where the genes with unknown functions and the genes with known functions and related to the disease are included in the predetermined number of features included in the pattern, the diagnosis support device corrects the pattern weight to become larger as the number or ratio of the genes with unknown functions included in the pattern is larger. Therefore, it is possible to extract the diagnostic criterion candidate that can also deal with unknown genes that have been difficult to appear as features in the past.

Note that, in the above-described embodiment, an example of genetic diagnosis has been described. However, the application of the disclosed technology is not limited to this. The disclosed technology can be applied to a case of predicting a diagnosis result based on a combination of a plurality of features and a diagnostic criterion. For example, the disclosed technology can be applied to medical diagnosis other than genes, and a case of diagnosis of the presence or absence of abnormalities based on sensing data such as image data.

Furthermore, in the above-described embodiment, a case of correcting the pattern weight calculated based on the rule weight, based on the number or ratio of the genes with unknown functions included in the pattern, has been described. However, correction of a pattern is not indispensable. Note that it is effective to correct the pattern weight as in the above-described embodiment in a case of adding the gene with an unknown function to the diagnostic criterion.

Furthermore, while a mode in which the diagnosis support program is stored (installed) in the storage unit in advance has been described in the embodiment described above, the embodiment is not limited to this. The program according to the disclosed technology may be provided in a form stored in a storage medium such as a compact disc read only memory (CD-ROM), a digital versatile disc read only memory (DVD-ROM), or a universal serial bus (USB) memory.

### REFERENCE SIGNS LIST

10 Diagnosis support device
12 Generation unit
14 Acquisition unit
16 Determination unit
18 Output unit
22 Sample data set
24 Rule set
40 Computer
41 CPU
42 Memory
43 Storage unit
49 Storage medium
50 Diagnosis support program

## Claims

1. A diagnosis support program that causes a computer to execute a process, the process comprising:
acquiring a set of rules, each rule represented by a combination of one or more features and generated by machine learning by using a training data set that includes a feature indicated by a sample as a diagnosis target and a feature indicated by a sample as a non-diagnosis target, and the each rule being associated with a first weight for the diagnosis target;
determining, for each pattern that includes a predetermined number of features, a second weight based on the first weight associated with the rule that includes the feature included in the pattern; and
outputting the pattern with the determined second weight that is equal to or greater than a predetermined value.

2. The diagnosis support program according to claim 1, wherein the rule is generated by the machine learning that assigns a degree of contribution to a diagnosis result of either the diagnostic target or the non-diagnosis target for each exhaustive combination of features indicated by the sample.

3. The diagnosis support program according to claim 1 or 2, wherein the training data set is a set of training data in which a value obtained by binarizing each feature of the features indicated by the sample is associated with a label that indicates whether the sample is the sample as the diagnosis target or the sample as the non-diagnosis target.

4. The diagnosis support program according to any one of claims 1 to 3, wherein the determining includes determining a total value of the first weights each associated with each of the rules that include the features included in the pattern as the second weight.

5. The diagnosis support program according to any one of claims 1 to 4, wherein,
in a case where the feature is a feature according to an expression level of a gene, and in a case where the predetermined number of features included in the pattern includes a gene with an unknown function and a gene with a known function,
the determining includes correcting the second weight to make the second weight larger as a number or a ratio of the genes with unknown functions included in the pattern is larger.

6. A diagnosis support device comprising:
an acquisition unit configured to acquire a set of rules, each rule represented by a combination of one or more features and generated by machine learning by using a training data set that includes a feature indicated by a sample as a diagnosis target and a feature indicated by a sample as a non-diagnosis target, and the each rule being associated with a first weight for the diagnosis target;
a determination unit configured to determine, for each pattern that includes a predetermined number of features, a second weight based on the first weight associated with the rule that includes the feature included in the pattern; and
an output unit configured to output the pattern with the determined second weight that is equal to or greater than a predetermined value.

7. The diagnosis support device according to claim 6, wherein the rule is generated by the machine learning that assigns a degree of contribution to a diagnosis result of either the diagnostic target or the non-diagnosis target for each exhaustive combination of features indicated by the sample.

8. The diagnosis support device according to claim 6 or 7, wherein the training data set is a set of training data in which a value obtained by binarizing each feature of the features indicated by the sample is associated with a label that indicates whether the sample is the sample as the diagnosis target or the sample as the non-diagnosis target.

9. The diagnosis support device according to any one of claims 6 to 8, wherein the determination unit determines a total value of the first weights each associated with each of the rules that include the features included in the pattern as the second weight.

10. The diagnosis support device according to any one of claims 6 to 9, wherein,
in a case where the feature is a feature according to an expression level of a gene, and in a case where the predetermined number of features included in the pattern includes a gene with an unknown function and a gene with a known function,
the determination unit corrects the second weight to make the second weight larger as a number or a ratio of the genes with unknown functions included in the pattern is larger.

11. A diagnosis support method executed by a computer, the diagnosis support method comprising:
acquiring a set of rules, each rule represented by a combination of one or more features and generated by machine learning by using a training data set that includes a feature indicated by a sample as a diagnosis target and a feature indicated by a sample as a non-diagnosis target, and the each rule being associated with a first weight for the diagnosis target;
determining, for each pattern that includes a predetermined number of features, a second weight based on the first weight associated with the rule that includes the feature included in the pattern; and
outputting the pattern with the determined second weight that is equal to or greater than a predetermined value.

12. The diagnosis support method according to claim 11, wherein the rule is generated by the machine learning that assigns a degree of contribution to a diagnosis result of either the diagnostic target or the non-diagnosis target for each exhaustive combination of features indicated by the sample.

13. The diagnosis support method according to claim 11 or 12, wherein the training data set is a set of training data in which a value obtained by binarizing each feature of the features indicated by the sample is associated with a label that indicates whether the sample is the sample as the diagnosis target or the sample as the non-diagnosis target.

14. The diagnosis support method according to any one of claims 11 to 13, wherein the determining includes determining a total value of the first weights each associated with each of the rules that include the features included in the pattern as the second weight.

15. The diagnosis support method according to any one of claims 11 to 14, wherein,
in a case where the feature is a feature according to an expression level of a gene, and in a case where the predetermined number of features included in the pattern includes a gene with an unknown function and a gene with a known function,
the determining includes correcting the second weight to make the second weight larger as a number or a ratio of the genes with unknown functions included in the pattern is larger.

16. A non-transitory computer-readable storage medium storing a diagnosis support program that causes a computer to execute a process, the process comprising:
acquiring a set of rules, each rule represented by a combination of one or more features and generated by machine learning by using a training data set that includes a feature indicated by a sample as a diagnosis target and a feature indicated by a sample as a non-diagnosis target, and the each rule being associated with a first weight for the diagnosis target;
determining, for each pattern that includes a predetermined number of features, a second weight based on the first weight associated with the rule that includes the feature included in the pattern; and
outputting the pattern with the determined second weight that is equal to or greater than a predetermined value.
